# FASCICULE DE BREVET EUROPEEN

(11) **EP 0 641 548 B1**
(45) Date de publication et mention de la délivrance du brevet: **19.11.1997**
(21) Numéro de dépôt: 94420235.7
(22) Date de dépôt: 30.08.1994
(51) Int. Cl.: A61B 17/60

(54) **Vis pour fixateur lombo-sacre**
Schraube für Lumbosakrale Fixationsvorrichtung
Screw for lumbo-sacral fixator

(30) Priorité: 01.09.1993 FR 9310581
(43) Date de publication de la demande: 08.03.1995
(73) Titulaire: TORNIER SA, 38330 Saint Ismier (FR)
(72) Inventeur: Tornier, Alain, F-38330 Saint-Ismier (FR)
(74) Mandataire: Monnier, Guy

(56) Documents cités:
- EP-A- 0 328 883
- DE-U- 9 302 700

## Description

La présente invention a trait à une vis pour la mise en place de fixateurs lombo-sacrés qui permettent une fusion des étages vertébraux endommagés lors d'une traumatologie de la colonne vertébrale.

On connaît, par exemple de EP-A-0 328 883, des vis pédiculaires qui comprennent généralement une tête pourvue d'une surface crantée qui se trouve dans un plan parallèle à celui de l'axe vertical de ladite vis. La tête est percée d'un trou taraudé et débouchant qui est perpendiculaire à l'axe de la vis. Une tige de liaison permettant de relier deux ou plusieurs vertèbres endommagées comporte à chaque extrémité libre un talon qui est percé d'un trou débouchant. L'une des faces du talon est crantée pour améliorer le contact avec celle de la vis pédiculaire. L'assemblage entre la tige et la tête de la vis est réalisé au moyen d'un boulon qui est serré à l'aide d'une clé.

On remarque que le serrage du boulon s'effectue perpendiculairement à l'axe vertical de la vis, ce qui complique considérablement le montage. En effet, l'espace libre prévu pour le passage de la clé de serrage est très réduit.

De plus, l'opérateur ne peut visser qu'1/6 de tour à la fois, car les becs de la clé viennent buter contre la paroi osseuse des apophyses transverses. En outre, en face de la tête du boulon se trouve l'épine dorsale de la vertèbre ce qui empêche l'opérateur d'utiliser un tournevis.

C'est à ces inconvénients qu'entend plus particulièrement remédier la présente invention.

La vis pédiculaire suivant l'invention comporte une partie filetée, une tête sphérique qui est pourvue d'une surface crantée inclinée par rapport à l'axe de la vis suivant un angle α d'environ 30 à 45° par rapport à l'axe vertical et un trou taraudé perpendiculaire à la surface crantée.

Selon une variante avantageuse de l'invention la partie filetée de la vis pédiculaire est pourvue d'une âme conique d'égale résistance comportant des filets tranchants sur toute la longueur du filetage. Selon une autre variante de l'invention, le trou taraudé débouchant est prévu débouchant et au milieu de la surface crantée.

Selon une autre variante de l'invention, la tête sphérique est percée d'un second trou taraudé qui communique avec le trou taraudé.

Selon une autre variante de l'invention, le second trou taraudé est apte à recevoir une vis de butée qui permet l'immobilisation en rotation d'un boulon vissé dans ledit trou taraudé.

Le dessin annexé, donné à titre d'exemple, permettra de mieux comprendre l'invention, les caractéristiques qu'elle présente et les avantages qu'elle est susceptible de procurer :
Fig. 1 est une vue perspective éclatée illustrant la vis pédiculaire suivant l'invention.
Fig. 2 est une vue montrant la mise en place de la vis dans le pédicule d'une vertèbre et l'immobilisation de la tige de liaison sur celle-ci.

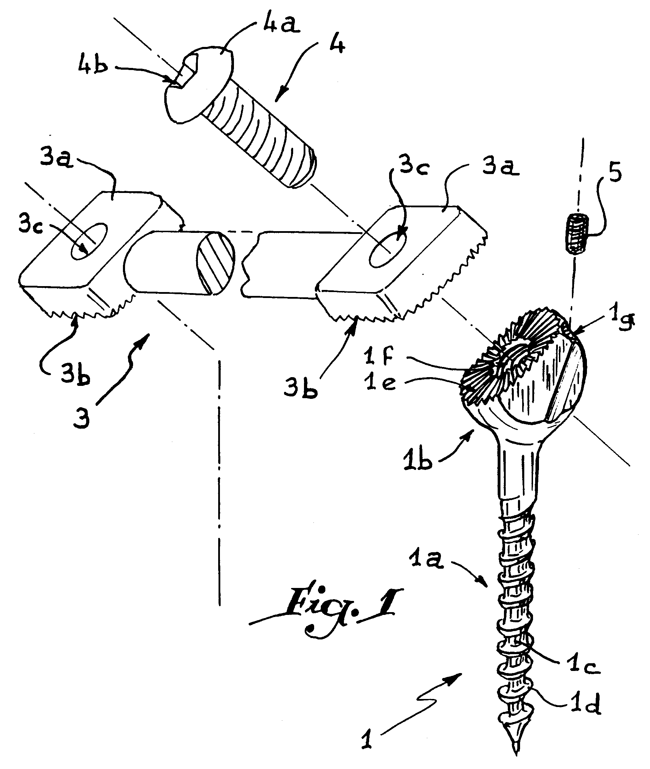

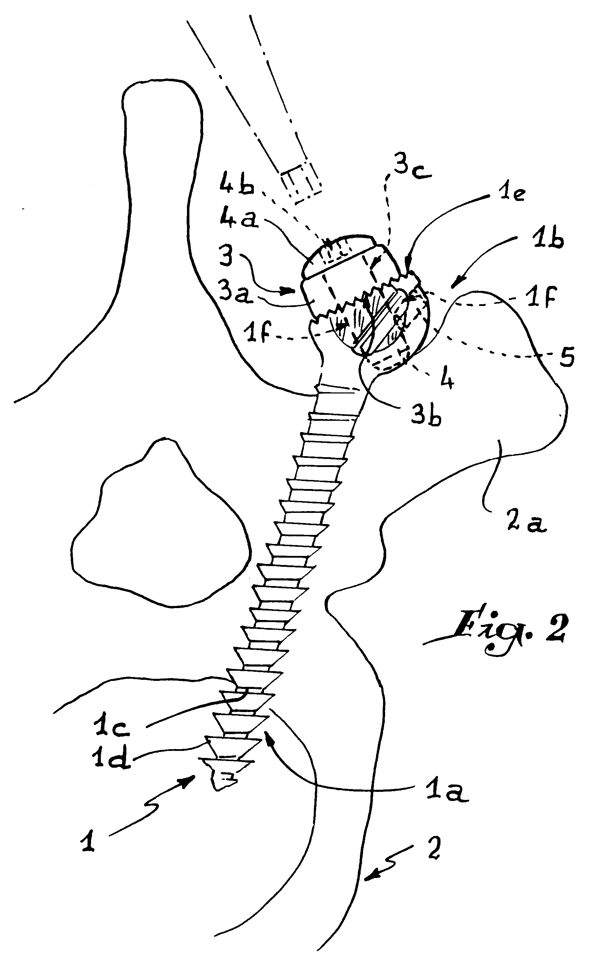

On a représenté en fig. 1 et 2 une vis pédiculaire 1 comportant une partie filetée 1a et une tête sphérique 1b.

La partie filetée 1a présente une âme 1c conique d'égale résistance et un filet tranchant 1d. Le filet 1d est prévu soit à profil conique et tranchant sur tout ou partie de sa longueur, soit droit et tranchant sur toute la longueur de la partie 1a.

La tête sphérique 1b de la vis pédiculaire 1 comporte une surface crantée 1e qui est inclinée par rapport à l'axe vertical de la vis d'un angle α d'environ 30 à 45°.

La tête sphérique 1b est percée au milieu de la surface crantée 1e et perpendiculairement à celle-ci d'un trou taraudé débouchant 1f.

Un autre trou 1g est percé suivant un axe parallèle à celui vertical de la vis pédiculaire 1, de manière qu'il communique avec le trou taraudé 1f.

La vis pédiculaire 1 est montée sur les pédicules 2a de chaque vertèbre 2 d'une colonne vertébrale (fig. 2). La surface crantée 1e est prévue pour recevoir les talons 3a d'une tige de liaison 3 d'un fixateur lombo-sacré. Chaque talon 3a présente une surface 3b correspondante à celle de la surface crantée 1e de la vis pédiculaire 1 pour que la liaison soit parfaite.

Un boulon 4 traverse l'alésage 3c ménagé dans chacun des talons 3a de la tige de liaison 3 pour venir se visser dans le trou taraudé 1f de la vis pédiculaire 1. Le boulon 4 comporte une tête 4a pourvue d'une empreinte à six pans creux 4b.

On remarque que l'inclinaison de la surface crantée 1e qui est relevée vers le haut permet à l'opérateur d'utiliser un simple tournevis pour serrer le boulon 4, dans le trou taraudé 1f. Lorsque le serrage de la tige de liaison 3 sur la tête de la vis pédiculaire 1 est réalisé à l'aide du boulon 4 l'opérateur introduit dans le trou 1g une vis de butée 5 qui permet d'immobiliser en rotation ledit boulon.

On note que l'utilisation d'un tournevis coopère avec un boulon 4 pourvue d'une empreinte hexagonale creuse 4b qui est noyée dans le corps de la vis, diminuant l'épaisseur de la tête et de gagner ainsi en encombrement.

On remarque que l'inclinaison de la surface crantée 1e décompose la rotation autour du boulon 4 en une rotation dans le plan parallèle à l'axe de la vis pédiculaire et en une rotation dans le sens perpendiculaire à l'axe de la vis.

La partie filetée 1a garantit une résistance optimale, tandis que le profil du filet 1d permet de s'ancrer solidement dans la partie osseuse du pédicule, évitant ainsi d'abîmer la corticale antérieure du corps vertébral.

## Revendications

1. Vis pour fixateur lombo-sacré de colonne vertébrale, comprenant une partie filetée (1a), caractérisée en ce qu'elle comprend une tête sphérique (1b) comportant une surface crantée (1e) inclinée par rapport à l'axe principal de la vis (1) d'un angle (α) d'environ 30 à 45° et un trou taraudé (1f) perpendiculaire à ladite surface crantée.

2. Vis suivant la revendication 1, caractérisée en ce que la partie filetée (1a) comporte une âme conique (1c) et un filet tranchant (1d) à profil conique ou droit sur toute sa longueur.

3. Vis suivant la revendication 1, caractérisée en ce que ledit trou taraudé et débouchant (1f) est prévu débouchant et au milieu de la surface crantée (1e).

4. Vis suivant la revendication 1, caractérisée en ce que la tête sphérique (1b) est percée d'un second trou taraudé (1g) qui communique avec ledit trou taraudé (1f).

5. Vis suivant la revendication 4, caractérisée en ce que ledit second trou taraudé est apte à recevoir une vis de butée (5) qui permet l'immobilisation en rotation d'un boulon (4) vissé dans ledit trou taraudé (1f).

## Claims

1. Screw for lumbo-sacral spine fixator, comprising a threaded part (1a), characterized in that it comprises a spherical head (1b) comprising a notched surface (1e) inclined with respect to the principal axis of the screw (1) by an angle (α) of about 30 to 45° and a tapped hole (1f) perpendicular to said notched surface.

2. Screw according to Claim 1, characterized in that the threaded part (1a) comprises a conical core (1c) and a cutting thread (1d) of conical or straight profile over the whole of its length.

3. Screw according to Claim 1, characterized in that said tapped, opening hole (1f) is provided to be open in the middle of the notched surface (1e).

4. Screw according to Claim 1, characterized in that the spherical head (1b) is pierced with a second tapped hole (1g) which communicates with said tapped hole (1h).

5. Screw according to Claim 4, characterized in that said second tapped hole is adapted to receive a stop screw (5) which allows a bolt (4) screwed in said tapped hole (1f) to be immobilized in rotation.

## Patentansprüche

1. Schraube für lumbosakrale Rückgratstütze mit einem Gewindeabschnitt (1a)
**gekennzeichnet durch**
einen kugelförmigen Kopf (1b), der eine verzahnte, zur Längsachse der Schraube (1) um einen Winkel (α) zwischen ungefähr 30 und 45° geneigte Oberfläche (1e) und eine senkrecht zu dieser verzahnten Oberfläche angeordnete Gewindebohrung (1f) aufweist.

2. Schraube nach Anspruch 1
**gekennzeichnet durch**
den Gewindeabschnitt (1a), der einen konisch zulaufenden Kern (1c) und ein schneidendes Gewinde (1d) mit einem über dessen gesamte Länge konisch oder gerade verlaufenden Profil aufweist.

3. Schraube nach Anspruch 1
**gekennzeichnet durch**
die einseitig offene Gewindebohrung (1f), die sich in der Mitte der verzahnten Oberfläche (1e) öffnet.

4. Schraube nach Anspruch 1
**gekennzeichnet durch**
den kugelförmigen Kopf (1b), der von einer zweiten Gewindebohrung (1g) durchsetzt ist, die mit der Gewindebohrung (1f) in Verbindung steht.

5. Schraube nach Anspruch 4
**gekennzeichnet durch**
die zweite Gewindebohrung (1g), die der Aufnahme einer Sicherungsschraube (5) dient, die die Verdrehsicherung eines in die Gewindebohrung (1f) geschraubten Gewindebolzens (4) gewährleistet.
